Europäisches Patentamt

European Patent Office

Office européen des brevets

Publication number: **0 287 012**
**A2**

# EUROPEAN PATENT APPLICATION

Application number: 88105722.8

Date of filing: 11.04.88

Int. Cl.⁴ **C12P 21/00 , C07K 15/00 ,
C12N 15/00 , G01N 33/577 ,
G01N 33/574 , G01N 33/68 ,
//C12N5/00,(C12P21/00,
C12R1:91)**

Priority: 17.04.87 JP 93233/87

Date of publication of application:
19.10.88 Bulletin 88/42

Designated Contracting States:
AT BE CH DE ES FR GB GR IT LI LU NL SE

Applicant: Eisai Co., Ltd.
6-10, Koishikawa 4-chome Bunkyo-ku
Tokyo 112(JP)

Inventor: Wakatsuki, Yoshio Sunresidence
Kinryo 317
Higashihori-kawaichijo agaru Kamikyo-ku
Kyoto-shi Kyoto(JP)
Inventor: Inada, Masami
15, Kamitakano-Sawabuchi Sakyo-ku
Kyoto-shi Kyoto(JP)
Inventor: Kudo, Hiroyuki
Freuden Kitaoji 202 42, Ichijoji-Jizomotocho
Sakyo-ku Kyoto-shi Kyoto(JP)

Representative: Hoffmann, Klaus, Dr. rer. nat.
et al
Hoffmann . Eitle & Partner Patentanwälte
Postfach 81 04 20 Arabellastrasse 4
D-8000 München 81(DE)

Anti-R-binder monoclonal antibody, process for preparing the same and application of the same.

An anti-R-binder monoclonal antibody is produced by hybridomas obtained by cell fusion of animal spleen cells immunized with an R-binder and myeloma cells followed by screening and specifically binds to an R-binder having a molecular weight of 60 to 80 K as determined by SDS polyacrylamide gel electrophoresis.

# ANTI-R-BINDER MONOCLONAL ANTIBODY, PROCESS FOR PREPARING THE SAME AND APPLICATION OF THE SAME

This invention relates to an anti-R-binder monoclonal antibody. a process for preparing the same and an application of the same. The present invention is particularly effective in assaying an R-binder for biochemical or medical purposes and thus utilized mainly in the field of clinical diagnosis.

( Statement of Prior Arts )

Intrisic factor (IF). transcobalamin II (TCII) and R-binders are important substances which bind to vitamin $B_{12}$ and take part in the transportation and storage thereof in vivo. In particular. it has been known that R-binders might selectively remove non-physiological materials from vitamin $B_{12}$ homologues. In addition, recently the behaviors of R-binders in the development of cancer have attracted attention. Thus it has been urgently required to clarify the mechanism thereof not only from the biochemical viewpoint but also from the medical one. As shown in references 3 to 11. some findings on R-binders have been reported hitherto. References are:

3) Stenman. J.H., Scand. J. Haematol.. 14.91-107 (1975)

4) Harcoullis. G., Progress in Gastroenterology (Academic Press) 1983, 133-172

5) Toskes. P. P.. ibid. 173-188

6) Allen. R. H.. Human Vitamine $B_{12}$ Transport proteins, Pregress in Hematology(ed. Elmer B. Brown) Vol. 9. Grune & Stratton, New York 1975

7) Waxman. S., Brit. J. Haematol.. Vol. 27. 229 (1974)

8) Carmel. R.. New Eng. J. Med., Feb. 6 pp282 (1975)

9) Waxman. S., Cancer Res.. 37, 1908-1914 (1977)

10) Gimsing. P., Scand. J. Haematol., 21. 243-249 (1978) and

11) Sheppard. K.. J. Clin. Pathol., 37, 1336-1338 (1984)

R-binders occur in blood and in other various bodily fluids. Immunohistological studies suggest that they occur in the digestive system including sialaden. glandula mucosa cells, conduit epithelium. esophagus glandula mucosa cells, hepatic bile duct epithelium, gallbladder epithelium, pancreatic duct epithelium, intestinal and intestinum columnar epithelium and goblet cells; in the respiratory system including superrespiratory tract epithelium and intrapulmonary bronchial glandula mucosa; and in the urinary system including renal papillary, prostatic epithelium, glandulae cervicales uteri, tubal epithelium. glandula mammaria epithelium and glandulae sudoriferae. It has been known that R-binders are proteins which widely occur as described above and bind to vitamin $B_{12}$.

However R-binders comprise a number of sugar proteins differing in molecular weight and isoelectric point from each other in practice. It has not been sufficiently established as yet how and what the R-binder should be assayed in order to obtain data valuable from the biochemical and medical point of view. This is because it has been impossible to assay selectively an R-binder which increases in a specified disease from various R-binders. For example. a conventional process for assaying R-binders by using $^{57}$co-cobalamin is not fully satisfactory. Namely. since R-binders bind to various vitamin $B_{12}$ homologues in vivo, the assay by using the $^{57}$Co-cobalamin can give only data on limited R-binders binding to cobalamin and those binding to vitamin $B_{12}$ homologues can not be assayed thereby.

Under these circumstances. the present inventor has attempted to apply R-binder assay to clinical diagnosis and to technically clarify an assay method and an assay reagent required therefor.

( Summary of the Invention )

As the result of extended studies, the present inventors have found that the above problem can be solved by using a novel anti-R-binder monoclonal antibody which is produced by hybridomas obtained by cell fusion of animal spleen cells immunized with an R-binder and myeloma cells followed by screening and specifically binds to an R-binder having a molecular weight of 60 to 80 K (as determined by SDS polyacrylamide gel electrophoresis), thus completing the present invention. The inventors have further succeeded in the preparation of an anti-R-binder monoclonal antibody which recognizes a specified isozyme of an R-binder by employing an interclonal subtraction method deviced by himself in said screening step. Accordingly it is the gist of the present invention to provide the abovementioned novel monoclonal antibody. a process for preparing the same, an immunoassay method by utilizing the same and an immunoassay reagent.

The invention provides an anti-R-binder monoclonal antibody and a process for preparation of the same. The anti-R-binder monoclonal antibody is produced from hybridomas obtained by immunizing an animal with an R-binder, conducting the cell fusion of animal spleen cells of the animal and myeloma cells and effecting screening to form the hybridomas. said antibody having specifically binded to an R-binder having a molecular weight of 60 to 80 K. determined according to the SDS polyacrylamide gel electrophoresis.

It is preferable that the screening step is conducted by selecting a hybridoma recognizing a single isozyme of an R-binder alone out of hybridomas derived from R-binders of different origins. by carrying out the sandwich immunoassay, in which aliquots of each hybridoma culture supernatant are added to solid phase plates prepared by binding said R-binder of different origins to a polyclonal antibody used as the primary antibody.

The anti-R-binder monoclonal antibody of the invention can be used for a method for assaying an R-binder by immunoassay and then as an immunoassay reagent for an R-binder. in particular for the diagnosis for a cancer of a digestive organ.

The R-binder according to the present invention may originate from any source such as saliva, milk. serum. gastric juice, bile and gastric or intestinal mucosa. Either a normal or cancerous source may be selected depending on the purpose. R-binders show a common antigen determinant regardless of the origins. Thus an antibody which recognizes said common antigen determinant is utilized in the present invention.

In the diagnosis of cancer. an antibody, which specifically recognizes an R-binder originating from a cancerous tissue and having an antigen determinant not present in a normal tissue, is prepared by subtraction to thereby enhance the specificity. An R-binder originating from a cancerous tissue has an antigen determinant common to, for example. that of an R-binder originating from saliva. Thus the former can be detected by using an antibody which recognizes the R-binder originating from saliva as well as the common antigen determinant. This fact is particularly obvious in assaying an R-binder having a molecular weight of 60 to 80 K (as determined by SDS polyacrylamide gel electrophoresis). Thus the R-binder to be assayed in the present invention has a molecular weight of 60 to 80 K. Any R-binder originating from any source may be used for immunizing animal cells. Generally an R-binder prepared from human saliva according to Allen's method (see ref. 1 and 2) may be employed therefor. Alternately an R-binder prepared from culture cells or culture supernatant of a human gastric cancer strain KATOH III, as will be described in Example 2 hereinafter, or those prepared from a resected carcinoma or normal portion of a resected stomach may be employed.

References are:

1) Allen.R.H., J.Biol. Chem., Vol.247, No. 23 7695-7701 (1972) and

2) Allen.R.H., ibid., Vol.249. No.22 7220-7227 (1974)

The animal to be immunized may be selected from. for example. rabbits and mice. Rabbits are preferable for obtaining an antiserum. while mice are preferable for obtaining immunized spleen cells. The immunization may be carried out in a conventional manner. For example. a mouse is immunized with an R-binder together with Freund's complete adjuvant. subjected to booster injection several times at intervals of two weeks and finally subjected to booster injection. and spleen cells of the animal are taken out three days thereafter.

As the myeloma cells. those developed for cell fusion, such as NS-1 or SP-2. may be employed.

As a fusing agent used in the cell fusion of spleen cells with myeloma cells, polyethylene glycol having an average molecular weight of approximately 1000 to 6000 has been used. In the present invention. polyethylene glycol of a molecular weight of 1500 may be employed therefor. The optimum concentration thereof preferably ranges from 40 to 50 % depending on the molecular weight. For example. 1 g of polyethylene glycol may be dissolved per m ℓ of a fetal calf serum-free RPMI-1640 medium and heated to 37°C for use.

The cell fusion may be carried out in a conventional manner as follows.

Spleen cells are mixed with myeloma cells at a ratio of 4 : 1 to 10 : 1 and the mixture is centrifuged and formulated into pellets. Then a fusing agent is added dropwise thereto at a ratio of 1 m ℓ per 1 to 2 $\times$ 10$^8$ cells. The obtained mixture is centrifuged to thereby remove the fusing agent and then suspended in an RPMI-1640 medium containing fetal calf serum, thus completing the fusion. Finally HAT selection is effected in a conventional manner to thereby separate the fused cells (hybridomas) from the parent cells.

Then the hybridomas are screened in an appropriate manner to thereby select those positive in the anti-R-binder production. In the Examples which will be described hereinafter. EIA, immunoprecipitation and fluorescent antibody methods are used in the screening.

In this screening step. it is convenient to employ an interclonal subtraction method established by the present inventor. Namely, a particular isozyme increasing in a certain disease is partially assayed in general by, for example, electrophoresis. However a troublesome procedure is required for preparing an antiserum for the particular isozyme. i.e., selective extraction of said isozyme alone followed by purification thereof. Therefore the present inventor has developed a process which comprises preparing a polyclonal antibody for the basic skeletone molecule, preparing solid phases of a number of immunogens differing in isozyme containing-pattern. screening a hybridoma for each immunogen and selecting a clone showing no crossing. The present inventor has named the above process "interclonal subtraction" It can be carried out in. for example. EIA in the following manner. First a polyclonal antibody is prepared by using an immunogen originating from saliva. By using this antibody as the primary antibody in sandwich EIA. various antigens originating from. for example. normal cells, cancerous cells or cancerous tissues are formulated into each a solid phase. Then each antigen is immunized and hybridoma corresponding thereto is separately prepared. The culture supernatant of the hybridoma is screened with the use of each solid phase plate obtained above. A hybridoma recognizing a plurality of antigens in common is removed and the residual ones are selected. Thus the purpose can be achieved without carrying out any troublesome procedure accompanying the conventional method. This subtraction method is available in preparing a monoclonal antibody for a particular isozyme. not only in the present invention but also in other cases.

Screening by immunoprecipitation and fluorescent antibody methods may be carried out in the following manner. Since an antigen R-binder would bind to cobalamin. $^{57}$Co-B$\cdot_2$ is added to antigens originating from various sources and allowed to bind thereto. Then the culture supernatants of the hybridomas to be screened are added thereto. The immune complexes thus formed are separated by using a solid phase antimouse antibody and the $\gamma$-ray thereof is counted. This is a simplified screening method available as assistant.

Among the positive hybridomas thus screened. those producing an antibody specifically binding to an R-binder having a molecular weight of 60 to 80 K (as determined by SDS polyacrylamide gel electrophoresis). which will be called the aimed antibody in the present invention, are selected by immunoprecipitation.

Namely, a hybridoma is deemed to be positive in the production of the aimed antibody in the present invention. when its radioautographic pattern shows a band corresponding to a molecular weight of 60 to 80 K by, for example, adding $^{125}$I-R-binder to the culture supernatant of said positive hybridoma in the lactoperoxidase method. subjecting the mixture to immunoprecipitation by using Protein A Sepharose and analyzing the same by 10 % SDS polyacrylamide gel electrophoresis. The molecular weight is not restrictly limited by the range as defined above. i.e.. 60 to 80 K and some variation is observed depending on the employed antigen. Since the molecular weight falls in this range in most cases. however, it is given as a standard from the practical viewpoint. The molecular weight of an R-binder falling into the abovementioned range proved to be 130 to 150 K by gel filtration with Sephadex G200. though it is mentioned herein only for reference.

Then the hybridomas producing the aimed antibody in the present invention are appropriately separated and cultured to thereby give monoclonal cell strains. This may be carried out by limiting dilution analysis. For example, a cell suspension is diluted with an RPMI-1640 medium supplemented with fetal calf serum and BALB.C mouse thymic cells, as a feeder, and then cultured on a tissue culture plate. The culture supernatants of wells showing multiplication are subjected to the abovementioned screening to thereby examine the presence of the antibody. This limiting dilution cloning may be repeated at least twice. In the Examples which will be described hereinafter. monoclonal cell strains 55-D and 42-C were obtained from an R-binder originating from saliva while strains WK-1. H-12 and B-3 were obtained from that originating from KATOH-III cells. These strains have been deposited with Fermentation Research Institute of the Agency of Industrial Science and Technology. Further the presence of immunogloblin in the monoclonal antibodies obtained by culturing these strains in vitro was examined. As a result. it was found that monoclonal antibodies produced by 55-D. 42-C, WK-1. H-12 and B-3 contained IgG1. IgG3. IgG3. IgG3 and IgG3. respectively.

The positive hybridomas thus obtained are multiplied by culturing in vitro or in vivo. A pure monoclonal antibody free from any other immunoglobulin can be obtained by culturing a positive hybridoma in vitro. Thus a positive hybridoma according to the present invention may be cultured in an appropriate medium such as an RPMI-1640 medium supplemented with fetal calf serum for an appropriate period of time. On the other hand, a large amount of a monoclonal antibody. though possibly contaminated with a small amount of other immunoglobulins, can be produced by culturing a positive hybridoma in vivo. For example, pristane (2,6,10,14-tetramethylpentadecane) is intraperitoneally administered to a BALB.C mouse. Two or more days thereafter, positive hybridomas according to the present invention are intraperitoneally administered to the animal and fixed and multiplied therein as an ascites fluid tumor within two or three weeks. Then the ascites fluid and/or serum of the animal are collected and the monoclonal antibody of the present invention is obtained therefrom through isolation and purification by, for example, salting out or chromatography. For example, the antibody activity of the collected ascites fluid is confirmed by immunoprecipitation. Then immunoglobulin is fractionated by chromatography and positive fractions are pooled.

The monoclonal antibody of the present invention specifically recognizes a protein antigen of an R-binder having a molecular weight of 60 to 80 K (as determined by SDS polyacrylamide gel electrophoresis). Thus an immunoassay method and an immunoassay reagent can be obtained by using the same, when said antigen is to be assayed for a biochemical or medical purpose. Immunoassay includes. e.g.. enzyme immunoassay, radioimmunoassay and passive hemagglutination. For example. enzyme immunoassay by the double antibody sandwich method can be carried out in the following manner.

The whole assay system comprises a solid phase. an R-binder antibody for coating the solid phase (the primary antibody), an anti-R-binder antibody (the secondary antibody), an enzyme-labeled antibody. a substrate, and a standard substrate or a specimen to be assayed. As the solid phase, wells of a microtiter plate for immunoassay may be employed. The primary antibody for coating said solid phase may be prepared from an antiserum. For example. rabbit antiserum immunized with an R-binder purified from human saliva is fractionated with ammonium sulfate and then IgG-fractionated with DEAE-Sepharose to thereby give a purified antibody. In Examples which will be described hereinafter. it is abbreviated to RabαhR. As the enzyme-labeled antibody, an appropriate one which is commercially available may be employed. For example. sheep $F(ab)_2$ antimouse IgG antibody labeled with horse radish peroxidase (HROP) (TAG; No. 4550) was used in Examples which will be described hereinafter. The substrate may be appropriately selected depending on the employed enzyme. Namely, when peroxidase is selected as the enzyme, o-phenylenediamine may be used as the substrate. As the standard antigen, a solution of a known concentration of an R-binder originating from an appropriate source such as saliva or gastric cancerous tissue culture cells may be employed. The assay may be carried out in a conventional enzyme immunoassay manner through the double antibody sandwich method. More precisely, the standard antigen or the specimen to be assayed is added to a well coated with the primary antibody and incubated therein. Then the culture supernatant of positive hybridomas is added thereto as the secondary antibody and incubated therein. Further the enzyme-labeled antibody is added thereto and incubated therein. Finally the substrate is added thereto and incubated therein. After ceasing the reaction, the amount of the decomposed substrate is determined.

The assay reagent of the present invention is directly available in the assay method of the present invention and thus can achieve the same object as the assay method does. Namely. the assay reagent of the present invention comprises the monoclonal antibody of the present invention as an essential component. More particularly. it comprises the monoclonal antibody of the present invention optionally together with one or more components selected from among solid phase. the primary antibody. enzyme-labeled antibody. substrate and standard antibody. thus forming a kit. The solid phase may be provided in a state coated with the primary antibody or the labeled antibody may be divided into, for example. an enzyme and an antibody, if required. The kit may further comprise. for example. appropriate diluent, solution or reaction ceasing agent in order to facilitate the embodiment of the assay. Thus the present invention is not restricted thereby.

The assay method of the present invention is highly useful in practice in the field of clinical examination, since it can be readily carried out and makes it possible to simultaneously treat a number of samples. It is particularly useful in the examination of cancer of a digestive organ by screening the plasma of a patient with a high accuracy. However the assay method of the present invention is not restricted to the diagnosis of the above-mentioned cancer but widely applicable to any case wherein an R-binder is available as a tumor marker and the diagnosis of cancer can be achieved by monitoring the same.

( Brief Explanation of Drawings )

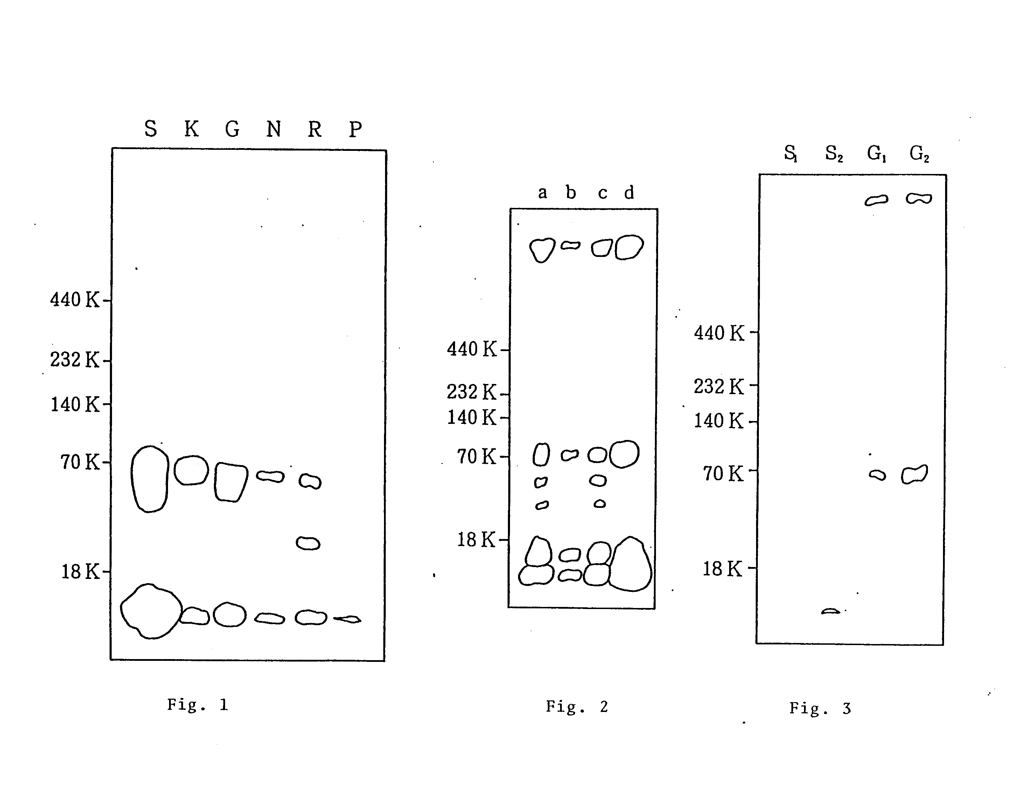

Figs. 1. 2 and 3 are model views of the results of radioautography through immunoprecipitation.

( Examples )

Example 1

i) Preparation of antigen:

This procedure was carried out according to reference 1. Namely, a cobalamin sepharose affinity column was prepared and 250 μg of an R-binder was purified from human saliva. Separately, human saliva was centrifuged at 10000 G for 30 minutes and the supernatant was filtered through a Watman N85 paper to thereby give a crude antigen.

ii) Preparation of antiserum antibody:

A rabbit was immunized with the purified R-binder prepared in i) to thereby give an antiserum. This antiserum was fractionated with ammonium sulfate and chromatographed with DEAE-cellulose to thereby purify an IgG fraction. The antibody thus obtained will be abbreviated to RabαhR hereinafter.

iii) Preparation of hybridoma:

A BALB.C mouse aged eight weeks was immunized with the purified R-binder obtained in i) together with Freund's complete adjuvant. Booster injection with the R-binder and Freund's complete adjuvant was effected several times at intervals of two weeks. Then the animal was finally subjected to booster injection with the purified R-binder and the spleen was taken out three days thereafter. Spleen cells were mixed with myeloma cells NS-1 and cell fusion was effected by adding polyethylene glycol of a molecular weight of 1500 to the mixture.

After effecting HAT selection, the aimed hybridomas were obtained.

iv) Screening.

The hybridomas thus obtained were subjected to the following ELISA screening (a) and immunoprecipitation screening (b) to thereby select hybridomas positive in the antibody production.

(a) ELISA

50 μℓ of RabαhR diluted 50-fold was introduced into a U-bottomed Falcon polyethylene flask and allowed to stand therein overnight. Then it was washed with 0.05 % Tween 20 PBS, which will be abbreviated to T-PBS hereinafter, thrice. 100 μℓ of 1 % bovine serum albumin/TBS, which will be abbreviated to B-TBS hereinafter, was added thereto and the resulting mixture was allowed to stand at room temperature for two hours and then washed with T-PBS. Subsequently 50 μℓ of the crude antigen originating from saliva as prepared in i) was added thereto and the resulting mixture was allowed to stand at room temperature for two hours. After washing with T-PBS, the culture supernatant of the hybridomas as obtained in iii) was added thereto and the mixture was allowed to stand at room temperature for two hours and then washed with T-PBS. 50 μℓ of HRPO-labeled goat F(ab)-₂αMIgG (γ L) (TAG; No. 4550) diluted 1500-fold was added thereto and the resulting mixture was allowed to stand at room temperature for two hours. Then it was washed with T-PBS and 200 μℓ of o-phenylenediamine substrate solution was added thereto. 30 minutes thereafter, 25 μℓ of 2 N $H_2SO_4$ was added thereto to thereby stop the reaction and $OD_{500}$ of the reaction mixture was determined.

(b) Immunoprecipitation

$^{57}Co$-B·₂ was added to the crude antigen originating from saliva as prepared in i) and the resulting mixture was allowed to stand at room temperature for 30 minutes and then dialyzed at 4°C for 24 to 48 hours to thereby remove unbonded $^{57}Co$-B·₂. To 20 μℓ of the $^{57}Co$-B·₂-labeled antigen, 100 μℓ of the culture supernatant of the hybridomas as obtained in iii) was added and the mixture was allowed to stand at 4°C overnight in an Eppendorf microtube. Then 40 μℓ of RabαMIg (A + G + M) (CPL; No. 3211-0231) diluted 100-fold was added thereto and the mixture was incubated in an end-to-end type shaker at room temperature for one hour. 40 uℓ of 1 % SAC (PANSORBIN, mfd. by Hoechst) was further added thereto and the resulting mixture was allowed to stand overnight at 4°C. Then it was centrifuged at 15000 rpm for ten minutes and the pellets thus obtained were counted. Mowe monoclonal anti-DNP antibody and normal mouse serum were employed as negative controls, while RabαhR diluted 10-and 100-folds were employed as positive ones.

v) Cloning:

The hybridomas screened in iv) were cloned by repeating limiting dilution analysis twice. As a result, anti-R-binder monoclonal antibody-producing cell strains 55-D and 42°C were obtained. These strains have been deposited with Fermentation Research Institute.

vi) Identification of the aimed antibody:

The presence of the aimed antibody of the present invention, which would recognize an R-binder of a molecular weight of 60 to 80 K, in the culture supernatant was examined by SDS polyacrylamide gel electrophoresis. Namely, the purified R-binder originating from saliva as prepared in i) was labeled with ¹²⁵I by the lactoperoxidase method. The labeled R-binder was added to the culture supernatant and Protein A Sepharose was further added thereto to thereby separate antigen/antibody complexes. Then SDS polyacrylamide gel electrophoresis and radioautography were carried out. As a result, each cell strain showed a band corresponding to a molecular weight of 60 to 80 K.

Separately each cell strain was cultured in vitro and the monoclonal antibody in the supernatant was precipitated by using ammonium sulfate and dialyzed against PBS. As a result, it was found that the supernatants obtained from 55-D and 42-C contained IgG1 and IgG3, respectively.

Example 2

i) Preparation of antigen:

A human gastric cancerous cell strain KATOH III was employed. This strain, which had been maintained in a medium comprising 45 % of an RPMI-1640 medium, 45 % of an MEM medium and 10 % of FCS, was washed with Hanks' solution thrice and cultured in a serum-free medium for seven to ten days. As a result, an R-binder having a molecular weight of 130 to 140 K (as determined by Sephadex G 200 gel filtration) was produced in the medium. This R-binder was also present in the cytoplasm at a concentration ten times as high as that achieved in the conventional maintenance medium. The culture supernatant was concentrated 10 to 100-fold with Amicon. On the other hand, the cells were sonicated in PBS containing 1 mM of PMSF and 0.001 % of thimerosal of a volume 50 times as much as the cells. The obtained mixture was centrifuged at 10000 G for 30 minutes and the supernatant was collected. These supernatants were combined together to give KIII antigen originating from gastric cancerous cells. The serum-free medium mentioned above was prepared by adding insulin, transferin and essential amino acids to an MEM or M199 medium to give the final concentrations of 5 $\mu$g/m$\ell$, 5 $\mu$g/m$\ell$ and 1 %, respectively.

ii) Preparation and screening of hybridoma:

The procedures of iii) and iv) of Example 1 were repeated, except that the crude antigen originating from saliva as used in (a) and (b) of iv) was replaced with the KIII antigen originating from gastric cancerous cells as prepared in above i).

iii) Cloning and identification of the aimed antibody:

The procedures of v) and vi) of Example 1 were repeated, except that the purified R-binder used in vi) was replaced by a crude antigen originating from a gastric cancerous tissue which will be described in Experimental Example 1 hereinafter. Thus anti-R-binder monoclonal antibody-producing cell strains WK-1, H-12 and B-3 were obtained. These strains have been deposited. Each strain contained a monoclonal antibody recognizing an R-binder of a molecular weight of 60 to 80 K (as determined by SDS polyacrylamide gel electrophoresis) and WK-1, H-12 and B-3 contained each IgG3.

Example 3

An example of the assay method according to the present invention will now be described with respect to an EIA system by the indirect sandwich method.

The Rah$\alpha$hR obtained in ii) in Example 1 was diluted 100-fold with PBS. 50 $\mu\ell$ portions of the solution were pipetted into wells of an enzyme immunoassay multiplate and incubated therein. After washing with T-PBS, 100 $\mu\ell$ of 1 % B-PBS was added thereto and the resulting mixture was allowed to stand at room temperature for one hour and washed with T-PBS. Then 50 $\mu\ell$ of a standard antigen or a specimen to be assayed was added thereto and the obtained mixture was allowed to stand at room temperature for two hours. After washing with T-PBS, 50 $\mu\ell$ of the culture supernatant of 55-D diluted 300-fold with 1 % B-PBS was added thereto and the resulting mixture was allowed to stand at room temperature for two hours. After washing with T-PBS, 50 $\mu\ell$ of HRPO-labeled goat F(ab)$_2$ $\alpha$MIgG ($\gamma$ L) (TAG: No. 4550) diluted 1500-fold with 1 % B-PBS was added thereto and the mixture was allowed to stand at room temperature for two hours and washed with T-PBS. 200 $\mu\ell$ of an o-phenylenediamine substrate solution was added thereto and the mixture was allowed to stand at room temperature for 15 to 30 minutes. Then 25 $\mu\ell$ of 2 N H$_2$SO$_4$ was added thereto to thereby cease the reaction and the OD$_{500}$ of the reaction mixture was determined.

Example 4

The monoclonal antibody of the present invention, which was prepared in the same manner as the one described in Example 1, was used as the assay reagent of the present invention. Separately, the monoclonal antibody of the present invention was combined with Rab$\alpha$hR prepared according to ii) of Example 1 to thereby give another assay reagent of the present invention in the form of a kit.

(5) Effects of the Invention:

To illustrate the effects of the present invention, the following Experimental Examples will be given.

Experimental Example 1

Sample:

RabαhR, 55-D, WK-1 and 42-C were prepared as antibody samples, while the crude antigen (S) originating from saliva as described in Example 1, the K III antigen (K) originating from gastric cancerous cells as described in Example 2, a crude antigen (G) originating from a gastric cancerous tissue, a crude antigen (N) originating from a normal gastric tissue, a patient serum (R) and bile (B) were prepared as antigen samples. G and N were prepared in the following manner. A resected stomach of Borrman III type (undifferentiated or moderately differentiated tabular adenocarcinomas) was employed. The carcinomas were observed exclusively on the inferior posterior wall of the stomach and the opposite part, i.e., the anterior wall was normal. A normal mucosa on the anterior wall was scraped onto a slide glass plate, while the cancerous tissue was collected by cutting with shears. To each material thus obtained, 0.25 M sucrose-Tris HCl (pH 7 - 6) weighing twice the moist substance was added and the resulting mixture was ground in a blender. 0.15 M NaCl containing 2 mM of PMSF, 1 mM of pepstatin and 0.001 % of methyl oleate, of a volume four times as much as that of the material, was further added thereto and the mixture was slowly stirred at 4°C for 48 hours and centrifuged at 20000 G for one hour. After collecting each supernatant, the sample G originating from the cancerous tissue and the sample N originating from the normal mucosa were obtained.

Method:

Each antigen sample was labeled with $^{125}I$, immunoprecipitated by using an appropriate antibody sample and subjected to SDS polyacrylamide gel electrophoresis followed by radioautography.

Result:

Figs 1, 2 and 3 show the results. Each figure shows the data obtained by radioautography wherein the ordinate refers to molecular weight. Fig. 1 shows a case wherein RabαhR alone was employed as the antibody sample and S, G, N, R and B were employed as the antigen samples. Fig. 2 shows a case wherein G alone was used as the antigen sample and the ascites fluid culture supernatant of 55-D (a), the culture supernatant of WK-1 concentrated 13-fold (b), the ascites fluid culture supernatant of WK-1 (C) and the ascites fluid culture supernatant of 42-C (d) were employed as the antibody samples. Fig.3 shows a case wherein the culture supernatant of WK-1 was employed as the antibody sample and S or G was used as the antigen sample. In Fig. 3, $S_2$ and $G_2$ show the results achieved when the antibody content of WK-1 was doubled compared with S. and G..

Fig. 1 shows a band common to all antigen samples at 60 to 80 K. Figs. 2 and 3 suggest that S and G show immunological crossing with each other at a molecular weight of 60 to 80 K and that 55-D recognizes the common antigen group while WK-1 exclusively recognizes the inherent one. Thus it has been found that WK-1, which selectively recognizes G, may be preferably employed in the detection of cancer, although 55-D, which recognizes a wide range of antigen determinants, may be also employed therefor, since the above purpose can be achieved by assaying the whole R-binder having a molecular weight of 60 to 80 K. Further the detection sensitivity can be improved by, for example, simultaneously using WK-1, H-12 and 55-D and the positiveness ratio may be improved by using WK-1 or H-12 alone.

Experimental Example 2

231 digestive disease plasma specimens, 54 hepatic pancreatic benign disease plasma specimens and 200 normal plasma specimens were assayed according to the method defined in Example 3. The mean (M) and standard deviation (SD) were determined from the data on the normal plasma group and the value of M + 2SD was employed as the cutoff value between positiveness and negativeness.

Result:

The results are as follows.
  A. Gastric cancer
    a) Positiveness ratio: 56/114, 49 %.
    b) Stage I: 17/37, 46 %; stage II: 9/14, 64 %;
stage III: 10/18, 56 %; stage IV: 15/31, 36 %;
and unknown: 5/14, 36 %.
    c) Tissue
highly differentiated: 6/11, 55 %;
moderately differentiated: 10/18, 56 %; and
lowly differentiated: 13/21, 62 %.

d) Metastasis

metastasized to liver: 6, 11, 55 %; and
unmetastasized: 12, 45, 27 %.

B. Pancreatic cancer including mamillary and terminal bileduct cancer
Positiveness ratio: 17, 64, 27 %.

C. Biliary cancer except pancreatic mamillary cancer Positiveness ratio: 7, 20, 35 %.
Colonic rectal cancer
Positiveness ratio: 8, 15, 53 %.

D. Hepatic cancer
Positiveness ratio: 8, 13, 62 %.

E. Esophageal cancer
Positiveness ratio: 0, 3, 0 %.

F. Hepatic pancreatic benign disease
Pseudopositiveness ratio: 13, 54, 24 %.

## Claims

1. An anti-R-binder monoclonal antibody which has been produced from hybridomas obtained by immunizing an animal with an R-binder, conducting the cell fusion of animal spleen cells of the animal and myeloma cells and effecting screening to form the hybridomas, said antibody having specifically binded to an R-binder having a molecular weight of 60 to 80 K, determined according to the SDS polyacrylamide gel electrophoresis.

2. A process for preparing an anti-R-binder monoclonal antibody, which comprises the steps of immunizing an animal with an R-binder, conducting the cell fusion of animal spleen cells of the animal and myeloma cells and effecting screening to form the hybridomas, said antibody having specifically binded to an R-binder having a molecular weight of 60 to 80 K, determined according to the SDS polyacrylamide gel electrophoresis.

3. A process claimed in Claim 2, which comprises a further step for selecting a hybridoma recognizing a single isozyme of an R-binder alone out of hybridomas derived from R-binders of different origins, by carrying out the sandwich immunoassay, in which aliquots of each hybridoma culture supernatant are added to solid phase plates prepared by binding said R-binder of different origins to a polyclonal antibody used as the primary antibody.

4. A method for assaying an R-binder by immunoassay which comprises the step of using an anti-R-binder monoclonal antibody as defined in Claim 1.

5. A method according to Claim 4, which is effected for the diagnosis of a cancer of a digestive organ.

6. An immunoassay reagent for an R-binder which comprises the anti-R-binder monoclonal antibody as defined in Claim 1.

7. Use of the immunoassay reagent as defined in Claim 6 as a diagnostic agent for a cancer of a digestive organ.

8. An anti-R-binder monoclonal antibody having binding specificity to an R-binder having a molecular weight determined according to the SDS polyacrylamide gel electrophoresis of 60 to 80 K.

0 287 012

S   K   G   N   R   P

440 K -

232 K -

140 K -

70 K -

18 K -

Fig. 1

a   b   c   d

440 K -

232 K -
140 K -

70 K -

18 K -

Fig. 2

S₁  S₂  G₁  G₂

440 K -

232 K -

140 K -

70 K -

18 K -

Fig. 3